# EUROPEAN PATENT SPECIFICATION

(11) **EP 0 269 246 B1**
(45) Date of publication and mention of the grant of the patent: **27.01.1993**
(21) Application number: 87309273.8
(22) Date of filing: 20.10.1987
(51) Int. Cl.: A61N 1/30

(54) **Novel plaster structure for iontophoresis**
Iontophorese-Pflaster
Emplâtre pour ionothérapie

(30) Priority: 20.10.1986 JP 250364/86
(43) Date of publication of application: 01.06.1988
(73) Proprietor: YAMANOUCHI PHARMACEUTICAL CO., LTD., Tokyo 103 (JP)
(72) Inventor: Konno, Yutaka, Yauzu-shi Shizuoka (JP); Mitomi, Mitsuo, Fujieda-shi Shizuoka (JP); Sonobe, Takashi, Fujieda-shi Shizuoka (JP); Yamaguchi, Shumpei, Tokyo (JP)
(74) Representative: Geering, Keith Edwin

(56) References cited:
- EP-A- 0 138 347
- US-A- 4 034 750

## Description

This invention relates to a novel plaster structure for iontophoresis.

Iontophoresis is a method of administering a medicament by passing electric current through the medicament to promote its absorption in an ionized state through the skin. The method usually involves attaching a medicament layer to one of a cathode or anode, mounting both electrodes on the skin at a definite spacing (with medicament layer against the skin), and passing an electric current between the electrodes from an electric current generator - see e.g., Japanese Patent Application (OPI) Nos. 156475/85, 188176/85, 31169/86, corresponding to EP-A-0 138 347 etc. The term "OPI" as used herein means an "unexamined published application".

A medicament which is positively ionized would be fixed to an anode, whereas a medicament which is negatively ionized would be fixed to a cathode. As the electrode which does not carry a medicament, a conductive gel layer permeated with sodium chloride is usually used. The absorption of medicament by iontophoresis generally increases as the electric current and voltage applied increase. However, when a high electric current of high voltage is passed through the skin for a long period, skin injury such as rubescence (burning) occurs. This sometimes reduces the absorption of medicament through the skin and makes difficult the adminstration of medicament by iontophresis. For solving these problems, an iontophoresis method employing a system of passing high frequency pulse electric current has recently been proposed - Japanese Patent Application (OPI) No. 31169/86.

The absorption of medicament by iontophoresis is influenced by the plaster structure containing the medicament as well as by the intensity and duration of the electric current. As conventional plaster structures for iontophoresis, there are known those wherein an electrode is covered by absorbent cotton impregnated with medicament, wherein a liquid containing medicament is infused into a tubular cap closely adapted to the skin and an electrode is connected to the cap, and wherein a conductive electrode layer and a medicament-containing conductive gel layer are simply laminated.

However, these plaster structures have sich disadvantages that the absorption of medicament is insufficient and decreases with the passage of time. Also, the first two plaster structures are inconvenient for handling and hence there are problems for their practical use.

The invention provides a plaster structure for iontophoresis comprising an electrode layer and a medicament-containing layer, characterised by a water supplying layer disposed between the electrode layer and the medicament-containing layer and a sealing layer over the electrode layer. The water supplying layer may comprise a water-containing self-adhesive lamina and/or a water-containing lamina. The plaster structure may have a capsule which has aqueous contents and is rupturable to impregnate the water-supplying layer with water.

Embodiments of the invention are illustrated, by way of example, by the accompanying drawings, in which :
Fig. 1 is a cross-sectional view showing the plaster structure of this invention obtained in Example 1;
Fig. 2 is a cross-sectional view showing the plaster structure of this invention obtained in Example 3;
Fig. 3 is a cross-sectional view showing the plaster structure of this invention obtained in Example 2;
Fig. 4 is a cross-sectional view showing a plaster structure equipped with an electrolyte capsule; and
Fig. 5 shows plasma calcium level versus time in guinea pigs after subcutaneous and iontophoretic administration.

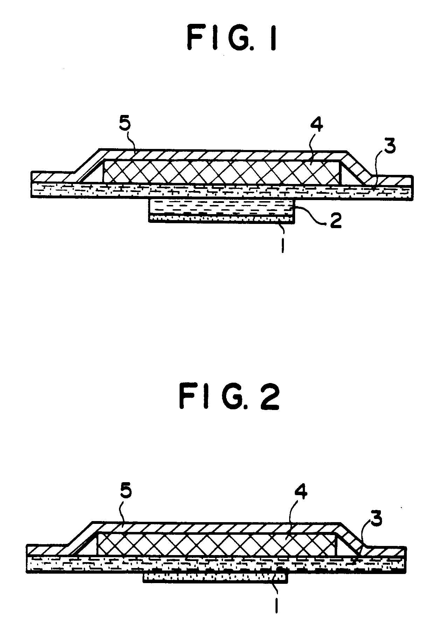

In Fig. 1 to Fig. 4 numeral 1 is a medicament-containing layer, 2 a water-containing lamina, 3 a water-containing self adhesive lamina, 4 an electrode, 5 a sealing cover, and 6 (Fig. 4) a water or electrolyte solution capsule.

The medicament-containing layer 1 is a thin layer composed of an element having high skin adhesivity and electric conductivity containing a medicament.

As the skin-adhesive material, a hydrophilic resin or a high mol.wt.compound can be used. As the hydrophilic resin, there are acrylic resins such as polyacrylamide, polyacrylic acid, and the alkali metal salts or esters thereof, vinylic resins such as polyvinylpyrrolidone, polyvinyl alcohol, polyvinyl ethyl ether, and the copolymers thereof, natural polysaccharides such as traganth gum, caraya gum, etc. Also, as the high molecular compound, there are methyl cellulose, hydroxypropyl cellulose, hydroxypropylmethyl cellulose, hyaluronic acid, and the alkali metal salts thereof.

For preparing the medicament-containing layer using the aforesaid material, the material is kneaded with a solution of medicament and compounded with water; with polyethylene glycol, propylene glycol, glycerol, etc., as soft plasticizer; and with sodium chloride, sodium carbonate, phosphoric acid, sodium citrate, etc., as electrolyte for imparting electric conductivity.

As a liquid for dissolving the medicament, a buffer liquid of phosphoric acid, citric acid, etc., is usually used but an aqueous solution of organic amine or mineral acid (such as hydrochloric acid,etc.)or an aqueous citric acid solution, etc., may be used singly or in combinations thereof according to the nature of a medicament.

Also, as a medicament-containing layer, a material such as cotton, filter paper, membrane filter, etc., impregnated with a medicament-containing solution having electric conductivity can be used.

The medicament-containing layer is dimensionally stable and is spread in film form or sheet form. The thickness thereof is preferably from 0.1 mm to 1.0 mm, particularly preferably from 0.2 mm to 0.5 mm. If it is too thick, it sometimes happens that good absorption of medicament through the skin is not obtained.

In regard to the concentration of medicament in the medicament-containing layer, the application of concentration gradient give good results; a medicament-containing layer of laminate layer type with the lamina which is brought into contact with the skin having the lowest concentration of medicament and laminae of increasing concentration being disposed thereover in succession to provide a concentration incline, can give stable and high absorption through the skin.

There is no particular restriction on the size (area) of the medicament-containing layer but the area is suitable from about 1/2 to about 1/3 of the area of the water-containing self adhesive layer 3.

As the medicament, an ionic medicament having a small distribution coefficient for oil is mainly used. Examples thereof are as in the following medicament groups although the invention is not limited to these medicaments.

### Antitussive Expectorant Agent

Sodium chromoglycate and Ketotifen fumarate

### Bronchodilator

Formoterol fumarate

### Analgesics

Narbuphine hydrochloride, Pentazocine lactate, Diclofenac sodium.

### Cardiac

Dopamine hydrochloride.

### Tranquilizers

Perphenazine and Phenothiazine.

### Antibiotics

Cefotetan disodium, Dibekacin sulfate, Amikacin sulfate, Netilmicin sulfate, and Sisomicin sulfate.

### Cytostatics

Adriamycin, Mitomycin C, Breomycin hydrochloride, Lentinan, Picibanil, Vincristine sulfate, Cisplatin

### Circulating Function Improving Agent

Nicardipine hydrochloride.

### Cerebral metabolism activator

Nicametate citrate, Meclofenoxate hydrochloride, Lisuride maleate, Calcium homopantothenate

### Gout Treating Agent

Allopurinol

### Peptides

LHRH, Enkephalin, Endorphin, Interferon, Insulin, Carcitonin, TRH, NT-36 (chemical name: N-[[(s)-4-oxo-2-azetidinyl]carbonyl]-L-histidyl-L-prolinamide), Oxytocin, Liprecin, Vasopressin, Glucagon, Pituitary hormones (e.g., HGH, HMG, HCG, Desmopressin acetate, etc.,), Follicle luteoids.

The water supplying layer (composed of a water-containing lamina 2 and/or a water-containing self adhesive lamina 3) is disposed between the medicament-containing layer 1 and the electrode layer 4.

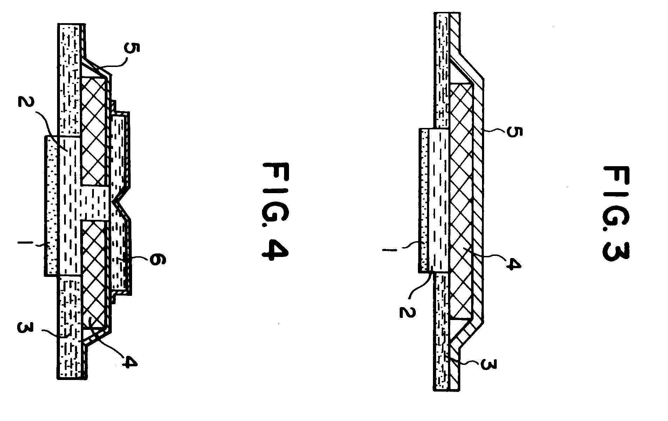

A water-containing lamina 2 may be of water retentive fibers such as cotton, sponge, cellulose triacetate, etc.; and a water-containing self adhesive lamina 3 may be of hydrophilic resin or high molecular wt. compound (as described hereinbefore) with electric conductivity. The water-containing lamina 2 and the water-containing self adhesive lamina may be used singly for the water supplying layer or both layers may be used as a laminate thereof. Furthermore, as shown in Fig. 3 and Fig. 4, the water-containing lamina 2 may be a central portion in contact with the medicament-containing layer 1 with the water-containing self adhesive lamina 3 disposed therearound.

The size (area) of the water supplying layer is the same as the area of the medicament-containing layer or larger than the latter area. When a water supplying layer of the aforesaid laminate type is employed, it is preferred that at least the water-containing self adhesive lamina 3 is larger than the area of the medicament-containing layer and covers the whole surface of the electrode layer, whereby direct contact of the electrode layer with the skin is prevented to reduce the occurence of skin injury. The thickness of the water-containing self adhesive lamina 3 is preferably from 1 mm to 5 mm from the view point of preventing skin injury.

The water-supplying layer may previously contain an electrolyte solution or may be filled with an electrolyte solution on the application thereof to the skin. For impregnating the water-supplying layer with an electrolyte solution, it is convenient to employ a capsule 6 containing water or electrolyte solution on the upper portion of the plaster structure, and a thin film such as aluminum foil, etc., disposed between the capsule 6 and the water-supplying layer being collapsed on application so as to impregnate the water-supplying layer with the electrolyte. The dry layer might contain electrolyte compound and the capsule 6 water.

Electrode layer 4 is an electric current dispersing material usually composed of conductive rubber, resin film, carbon film, or metal foil such as aluminum foil, etc. It is preferred that the area of the electrode is from 2 to 3 times the area of the medicament-containing layer. By employing the aforesaid construction, the current density per unit area can be reduced to reduce skin injury and accelerate the absorption of medicament through the skin.

The sealing cover 5 is a water impermeable skin adhesive sheet. The cover is usually composed of flannel or cotton cloth impregnated with vaseline or an adhesive plastics tape, etc. The sealing cover may cover the whole surface of the electrode layer and extending beyond of the electrode layer.

The plaster structure of this invention usually has a waterproof release liner on the side thereof to be brought into contact with the skin and is used for practical purpose in a sealed state. For a water-decomposable medicament, it is better that the medicament-containing layer 1 and the water-containing layer 2 are dry with a capsule 6 containing an electrolyte solution formed on the water-containing layer 2 as shown in Fig. 4; dry layer 1 and/or 2 might contain electrolyte compound and capsule 6 just water.

As described above, the plaster structure of this invention is novel in having a water supplying layer and a sealing cover; due to these layers, sufficient supply of water into the medicament-containing layer can be attained to give efficient absorption of the medicament through the skin. Also, the employment of a medicament-containing layer having a concentration gradient and the employment of the water-containing shelf adhesive lamina 3 over the whole surface of the electrode layer are useful for increasing the absorption of medicament and skin safety.

When using the plaster structure of this invention, the structure and a counter electrode are mounted on the skin with a spacing of at least few millimeters and a desired electric current is passed through the electrode layer 4 and the counter electrode by lead wires from an electric current generator.

The following are examples of the plaster structure of this invention.

### Example 1

A definite amount of a medicament was dissolved in a liquid, the solution was kneaded with polyacrylamide, and a medicament-containing layer 1 2.8 cm² in area and 0.4 mm thick was prepared. A water-containing lamina 2 composed of cotton cloth (2.8 cm² in area and 0.2 mm thick) impregnated with 400 µl of water and having electric conductivity was laminated to the medicament-containing layer 1. Further a circular water-containing self adhesive lamina 3 of polyacrylamide gel (8.5 cm² in area and 0.4 mm thick, containing 80% water) was laminated thereon. An electrode layer 4 having the same area as above was disposed on layer 3 and a sealing cover 5 composed of a sheet having an adhesive layer of natural rubber adhesive (made by Marusho Kinzoku Haku K.K.) was covered over the outer surface thereof to provide a plaster structure covered by the sealing cover 5 (see Fig. 1).

### Example 2

A circular hole 2.8 cm² in area was formed at the center of a circular polyacrylamide lamina (8.5 cm² in area and 0.4 mm thick, containing 80% water) and in the hole were mounted a medicament-containing layer 1 and a water-containing lamina 2 prepared as in Example 1. An electrode layer 4 and a sealing cover 5 were disposed thereon as in Example 1 to provide the plaster structure of Fig. 3.

### Example 3

This plaster structure was prepared in the same manner as Example 1 except that the water-containing lamina 2 was omitted (see Fig. 2).

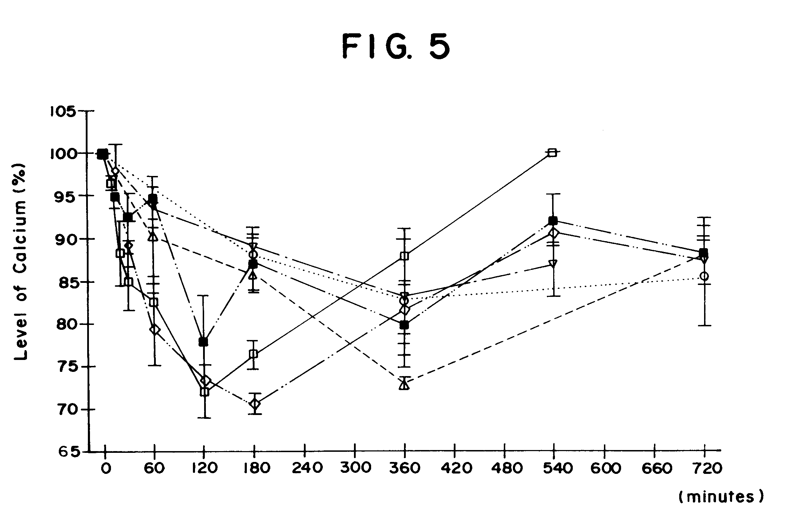

Using plaster structures of this invention prepared as above, the absorption of medicament by iontophoresis was measured under the following experimental conditions. The results are compared with those from conventional administration modes in Table 1 and Fig.5.

### Experimental Conditions:

- Medicament:: Salmon carcitonin (hereinafter referred to as sCT) having a molecular weight of about 3,400
- Animal:: Guinea pigs
- Measurement Method:: The reduction of plasma calcium concentration by the absorption of sCT through the skin was measured. The measurement of the plasma calcium concentration was perfomed using Calcium Meter Type CA-30 (made by Jokosha K.K.).
- Administration Method:: Experimental Examples 1 to 3 shown administration by plaster structures of this invention, and Comparison Examples 1 to 3 shown administration by subcutaneous injection, a solution cap method, and a conventional plaster (without the water supplying layer and sealing cover).

### Comparison Example 1 (Subcutaneous injection method, sCT 5 IU/kg)

After injection of sCT solution (an aqueous sCT solution of 0.0001 M citric acid) into the back portion of a guinea pig, the blood was taken at the times indicated in Fig.5 and the concentration of calcium was measured.

### Comparison Example 2 (Solution cap method, sCT 100 IU/kg)

In a Mcllvaine buffer of pH 4.0 was dissolved sCT to provide a solution of IU/ml of sCT. Then, about 2 ml of the solution was placed in an acrylic resin cap (2.8 cm² in area and 1 cm in height, equipped with an electrode at the inside of the cap) previously mounted on the skin of abdomen of a guinea pig and an electric current of 12 volts and 40 KHz was passed for 1 to 12 hours. In addition, as a cathode, an acrylamide gel sheet prepared by kneading with an aqueous sodium chloride solution was used. The blood was taken at the times indicated in Fig.5 and the concentration of calcium was measured.

### Comparison Example 3 (Conventional plaster assembly, sCT 100 IU/kg)

An sCT-containing gel sheet was prepared so that the dose of sCT to guinea pig became 100 IU/kg and was placed on a conductive silicone rubber side of an anode layer (2.8 cm² in area stainless steel foil/conductive silcone rubber). The anode layer was mounted on the skin of a guinea pig, an electric current of 5 mA and 40 KHz was passed therethrough, the blood was taken at the times indicated in Fig.5 and the concentration of calcium was measured.

### Experimental Example 1 (sCT 20 IU/kg)

An aqueous 1% gelatin solution of 0.0001 M citric acid was used for dissolving sCT and the plaster structure shown in Example 3 having the medicament-containing layer prepared by the aqueous solution of sCT so that the doses of sCT to guinea pig became 20 IU/kg was mounted on the skin of a guinea pig. An electric current of 10 mA and 40 KHz was passed therethrough for from 1 to 9 hours, the blood was taken at the times indicated in Fig.5 and the concentration of calcium was measured.

### Experimental Example 2 (sCT 20 IU/kg)

The plaster structure shown in Example 1 having the medicament-containing layer of Experimental Example 1 was mounted on the skin of a guinea pig. An electric current of 10 mA and 40 KHz was passed therethrough for from 15 minutes to 12 hours; blood was taken at the times indicated in Fig.5 and the concentration of calcium was measured.

### Experimental Example 3 (sCT 10 IU/kg)

The concentration of calcium was measured as in Experimental Example 2 using the plaster structure of Example 1 having the medicament-containing layer prepared so that the doses of sCT became 10 IU/kg.

### Results

The calcium level in the blood plasma at various times is shown in Fig. 5 and the relative reduction ratio of calcium to the intravenous injection of 5 IU/kg is shown in Table 1:

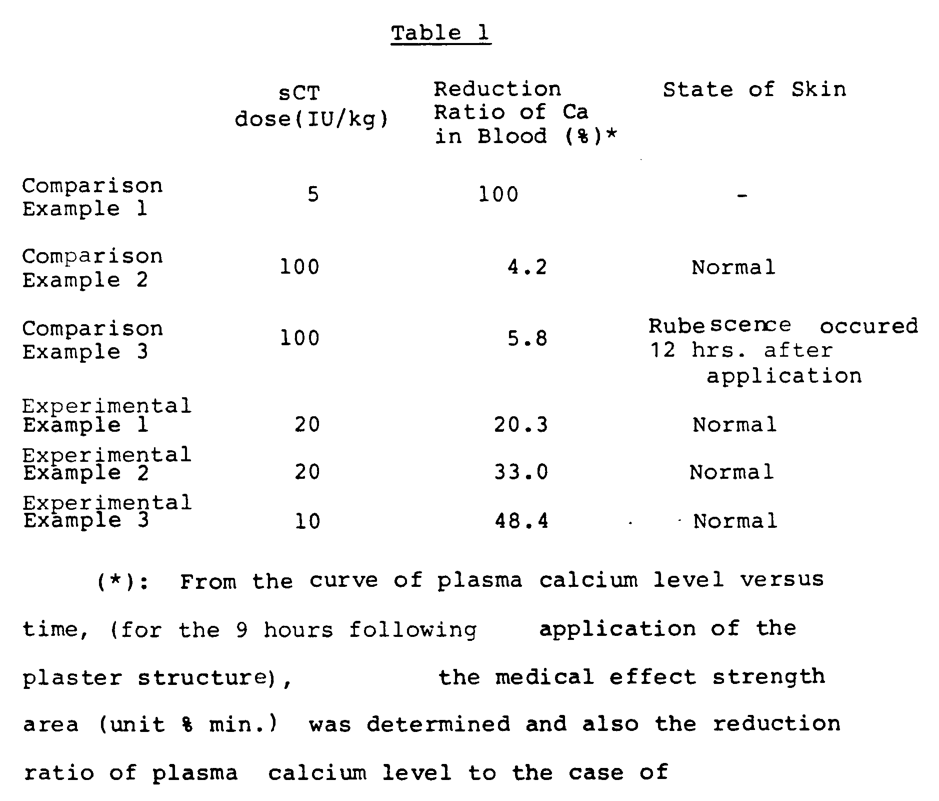

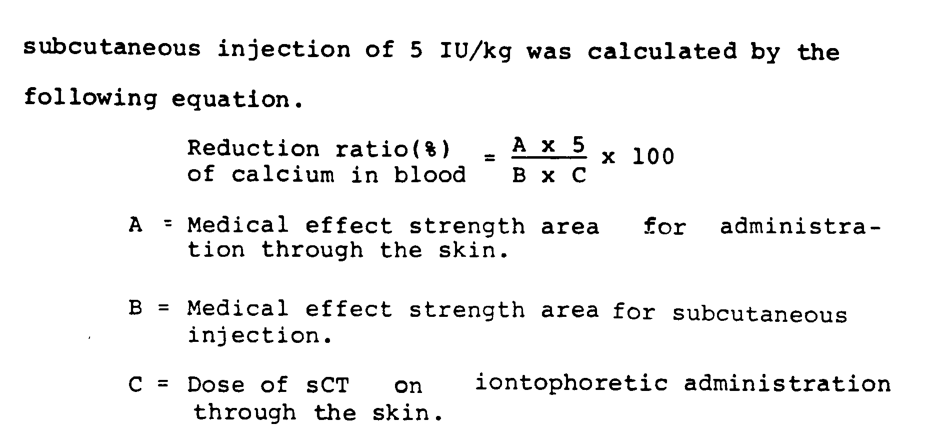

"Medical effect strength area" means the lowering effect (by iontophoresis or subcutaneous injection) on calcium in plasma when the initial amount of calcium in plasma is considered to be 100%. Thus it means the area of the curved line in the following graph :

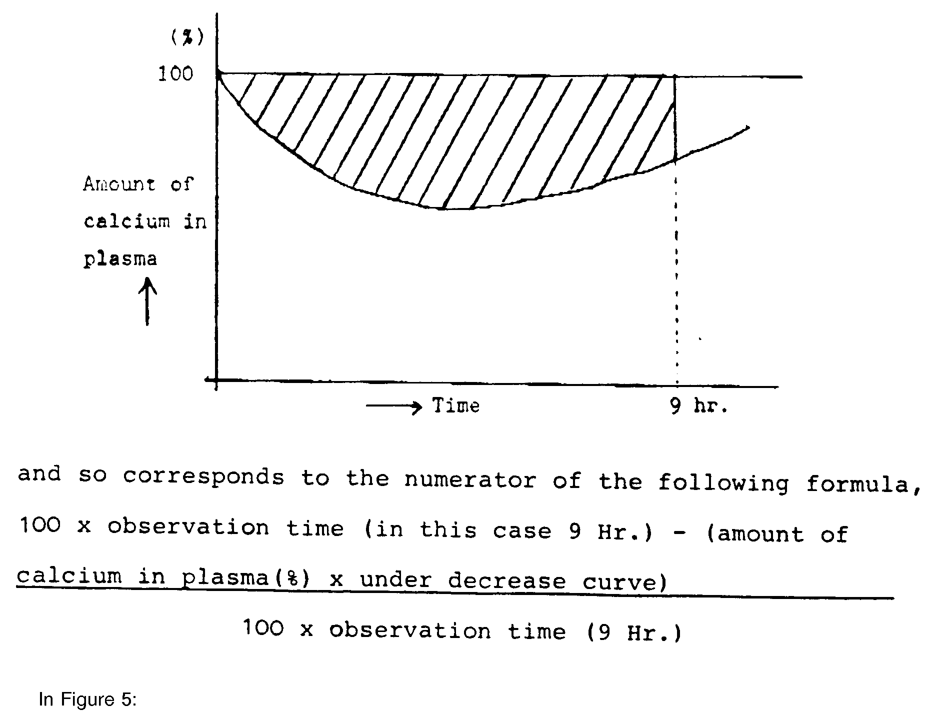

In Figure 5:

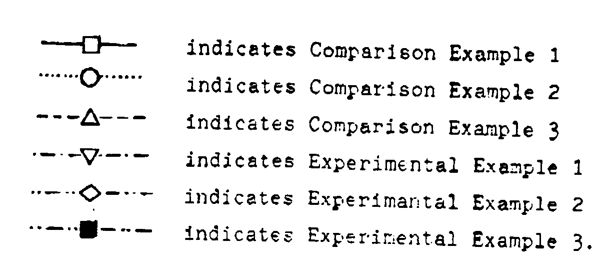

## Claims

1. A plaster structure for iontophoresis comprising an electrode layer (4) and a medicament-containing layer (1), characterised by a water supplying layer (2, 3) disposed between the electrode layer and the medicament-containing layer and a sealing cover (5) over the electrode layer.

2. A plaster structure according to claim 1 wherein the water supplying layer comprises a water-containing self adhesive lamina and/or a water-containing lamina.

3. A plaster structure according to claim 1 or 2 wherein the thickness of the medicament-containing layer is from 0.1 mm to 1.0 mm.

4. A plaster structure according to any preceding claim wherein there is a medicament concentration gradient in the medicament layer, the medicament concentration decreasing in the direction away from the water-supplying layer.

5. A plaster structure according to any preceding claim having a capsule which has aqueous contents and is rupturable to impregnate the water-supplying layer with water.

## Patentansprüche

1. Pflasterstruktur zur Iontophorese, umfassend eine Elektrodenschicht (4) und eine Arzneimittel-haltige Schicht (1), gekennzeichnet durch eine wasserliefernde Schicht (2, 3), die sich zwischen der Elektrodenschicht und der Arzneimittel-haltigen Schicht befindet, und eine dichtende Abdeckschicht (5) über der Elektrodenschicht.

2. Pflasterstruktur nach Anspruch 1, wobei die wasserliefernde Schicht eine wasserhaltige selbtklebende Schicht und/oder eine wasserhaltige Schicht umfaßt.

3. Pflasterstruktur nach Anspruch 1 oder 2, wobei die Dicke der Arzneimittel-haltigen Schicht 0,1 bis 1,0 mm beträgt.

4. Pflasterstruktur nach einem der vorangehenden Ansprüche, wobei ein Gradient der Arzneimittelkonzentration in der Arzneimittel-haltigen Schicht existiert, wobei die Arzneimittelkonzentration in Richtung von der wasserhaltigen Schicht weg abnimmt.

5. Pflasterstruktur nach einem der vorangehenden Ansprüche mit einer Kapsel, die einen wäßrigen Inhalt aufweist und die aufgebrochen werden kann, um die wasserliefernde Schicht mit Wasser zu imprägnieren.

## Revendications

1. Une structure d'emplâtre pour ionothérapie comprenant une couche d'électrode (4) et une couche contenant un médicament (1),
caractérisée par une couche d'amenée d'eau (2, 3) disposée entre la couche d'électrode et la couche contenant un médicament et un couvercle de scellement (5) au-dessus de la couche d'électrode.

2. Une structure d'emplâtre selon la revendication 1, dans laquelle la couche d'amenée d'eau comprend une lamelle autoadhésive contenant de l'eau et/ou une lamelle contenant de l'eau.

3. Une structure d'emplâtre selon la revendication 1 ou 2, dans laquelle l'épaisseur de la couche contenant un médicament est de 0,1 mm à 1,0 mm.

4. Une structure d'emplâtre selon une quelconque revendication précédente, dans laquelle il y a un gradient de concentration de médicament dans la couche de médicament, la concentration en médicament diminuant dans la direction d'écartement d'avec la couche d'amenée d'eau.

5. Une structure d'emplâtre selon une quelconque revendication précédente, ayant une capsule qui a un contenu aqueux et est fracturable pour imprégner d'eau la couche d'amenée d'eau.
